# EUROPÄISCHE PATENTSCHRIFT

(11) **EP 0 638 613 B1**
(45) Veröffentlichungstag und Bekanntmachung des Hinweises auf die Patenterteilung: **23.12.1998**
(21) Anmeldenummer: 94810459.1
(22) Anmeldetag: 04.08.1994
(51) Int. Cl.: C09B 5/62, G03G 5/06

(54) **Perylenamidinimid-Farbstoffe, ein Verfahren zu deren Herstellung und deren Verwendung**
Perylene amidine-imide dyes, a process for their production and their use
Colorants perylène amidine-amide, procédé pour leur préparation et leur utilisation

(30) Priorität: 13.08.1993 CH 2413/93
(43) Veröffentlichungstag der Anmeldung: 15.02.1995
(73) Patentinhaber: Ciba Specialty Chemicals Holding Inc., 4057 Basel (CH)
(72) Erfinder: Langhals, Heinz, Prof. Dr., D-85521 Ottobrunn (DE)

(56) Entgegenhaltungen:
- EP-A- 0 143 979
- EP-A- 0 447 826
- DE-A- 3 017 935
- DE-A- 3 209 424
- DE-A- 4 037 735
- US-A- 4 336 383
- US-A- 4 714 666
- US-A- 4 968 571
- CHEMISCHE BERICHTE, 116. Jahrgang, Heft 8, CHEMIE-Verlag, Weinheim IVAN LUKAC et al. "Dar- stellung und Fluoreszenz- verhalten von 2,3,4,4a,10a, 11,12,13-Octahydro-1,4a,10a, 14-tetraazaviolanthron- -Derivaten" Seiten 3524-3528
- BULLETIN OF THE CHEMICAL SOVIETY OF JAPAN, vol. 25, 1952, THE CHEMICAL SOCIETY OF JAPAN, Tokyo TOSHIO MAKI et al. "Vat Dyes of Acenaphthene Series IV. Condensation of Perylen- etetracarboxylic Acid Anhydride with o-Phenylene- diamine" Seiten 411-413
- CHEMISTRY LETTERS, no. 2, February 1979, CHEMICAL SOCIETY OF JAPAN, YUKINORI NAGO et al. "Synthesis of Unsymmetrical Perylenebis(Dicarboximide) Derivatives" Seiten 151-154

## Beschreibung

Die Erfindung betrifft bestimmte, mit sekundären Alkylresten oder mit alkylsubstituierten Phenylresten am Imidstickstoff substituierte Perylenamidinimide, ein Verfahren zu deren Herstellung durch Umsetzung der entsprechenden N-substituierten Perylen-3,4:9,10-tetracarbonsäure-monoanhydrid-monoimide mit Diaminen, und deren Verwendung, beispielsweise für die Massefärbung von hochmolekularem organischen Material, als Fluoreszenzfarbstoffe oder als photoleitfähige Substanzen.

Die seit langer Zeit bekannten, schwer löslichen Perylenfarbstoffe, Perylen-3,4:9,10-tetracarbonsäurebisimide, **2**, sind aus dem im Handel erhältlichen Perylen-3,4:9,10-tetracarbonsäurebisanhydrid **1** leicht zugänglich und werden hauptsächlich als Pigmente eingesetzt.

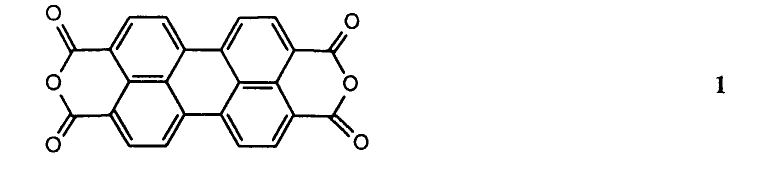

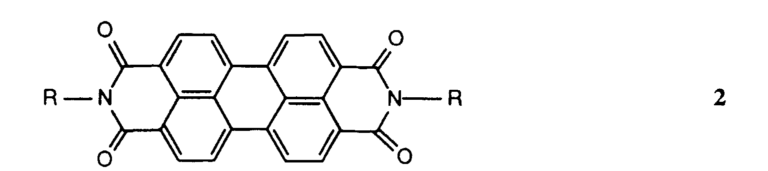

Auf der Basis dieses Chromophors können aber auch hoch lichtechte Fluoreszenzfarbstoffe entwickelt werden, wenn die beiden Stickstoffatome der Imid-Gruppen mit löslichkeitssteigernden Substituenten R, wie z.B. dem 2,5-Di-tert-butylphenyl- oder dem 1-Hexylheptyrest (siehe z.B. S. Demmig, H. Langhals, Chem. Ber. 1988, **121**, 225; H. Langhals, S. Demmig, T. Potrawa, Journal f. prakt. Chemie, 1991, **333**, 733), versehen sind. Während viele Eigenschaften der Perylenfarbstoffe, wie die Löslichkeit in organischen Lösungsmitteln, in weiten Grenzen durch die Reste R beeinflußt werden können, ist eine Veränderung der UV/Vis-Spektren auf diesem Wege kaum möglich (A. Rademacher, S. Märkle, H. Langhals, Chem. Ber. 1982 **115**, 2927). Eine Kern-Substitution der Farbstoffe ist zwar prinzipiell möglich, sie bereitet jedoch wegen sterischer Wechselwirkungen häufig Probleme.

Eine weitere Möglichkeit, die Spektren zu verändern besteht darin, Carbonylgruppen der Perylenfarbstoffe durch die verwandten Imino-Gruppen zu ersetzen. Eine Ersatz jeweils einer Carbonylgruppe der beiden Carbonsäureimid-Ringe führt zwar zu Farbstoffen, deren Absorption längerwellig verschoben ist, ihre Löslichkeit in organischen Lösungsmitteln ist allerdings verhältnismäßig gering, und die Fluoreszenzquantenausbeuten sind kleiner als die der Perylenfarbstoffe (I. Lukac, H. Langhals, Chem. Ber. 1983, **116**, 3524).

Schlüssel-Verbindungen für die Darstellung der genannten Farbstoffe sind die Perylen-3,4:9,1 O-tetracarbonsäure-3,4-anhydrid-9,10-imide **3**, 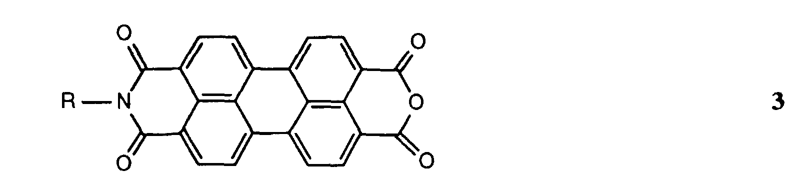 die nach einem neuen Verfahren durch eine partielle, alkalische Verseifung der Bisimide zugänglich werden (H. Kaiser, J. Lindner, H. Langals, Chem. Ber. 1991, **124**, 529). Das letztere ermöglicht auch die Einführung löslichkeitssteigernder Reste, wie z.B. der 1-Hexylheptyl-Rest.

Gegenstand vorliegender Erfindung sind Perylenamidinimide der Formel I 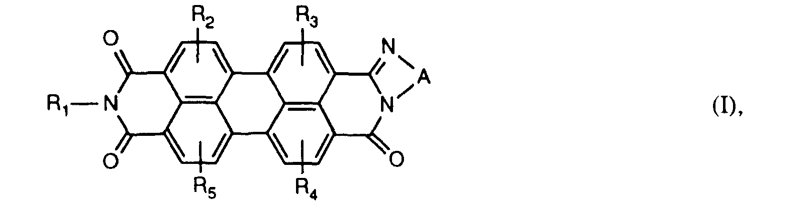 worin R₁ einen sekundären C₇-C₄₁-Alkylrest oder einen Rest der Formel II bedeutet 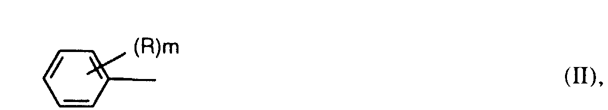 worin R ein verzweigter C₃-C₈-Alkylrest ist und m 1, 2 oder 3 bedeutet;
A für C₅-C₇-Cycloalkylen, Phenylen, Naphthylen, Pyridylen, einen höher kondensierten aromatischen carbocyclischen oder heterocyclischen Rest oder einen zweiwertigen Rest der Formel III, IV oder V steht 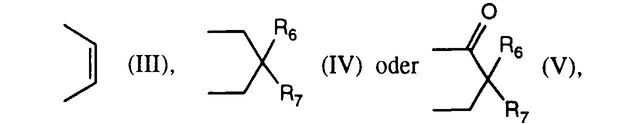 wobei sowohl R₁ als auch A durch Halogen, Alkyl, Cyano oder Nitro substituiert sein können;
R₂ bis R₅ unabhängig voneinander H, Alkyl, Aryl, Heteroaryl, Halogen, Cyano, Nitro, -OR₈, -COR₈, -COOR₈, -OCOR₈, -CONR₈R₉, -OCONR₈R₉, -NR₈R₉, -NR₈COR₉, -NR₈COOR₉, -NR₈SO₂R₉, -SO₂R₈, -SO₃R₈, SO₂NR₈R₉ oder -N=N-R₈ bedeuten; und R₆ bis R₉ unabhängig voneinander C₁-C₄-Alkyl, Phenyl oder 4-Tolyl sind.

Die erfindungsgemässen Verbindungen stellen Farbstoffe mit neuen Chromophoren dar, die im Vergleich zu den Perylentetracarbonsäure-Bisimiden lägerwellig absorbieren und stark fluoreszieren. Im Vergleich zu den eingangs erwähnten, aus Chem. Ber. 1983, **116**, 3524 bekannten Perylentetracarbonsäure-B isamidin-Derivaten weisen die neuen Amidinimide wesentlich höhere Fluoreszenzquantenausbeuten auf. Zudem kann bei den erfindungsgemässen Verbindungen das Absorptionsmaximum durch Variierung des A Restes nach Wunsch gesteuert werden. Die Amidinimide der Formel I zeichnen sich durch eine für diesen Typ von Verbindungen hohe Löslichkeit in organischen Lösungsmitteln sowie durch ganz vorzügliche Lichtechtheit aus.

Einige wenige Perylentetracarbonsäure-Amidinimide sind bereits in der Literatur erwähnt. So beschreiben T. Maki und H. Hashimoto im Bull. Chem. Soc. Jpn., 1952, **25**, 411, die Kondensation von Perylentetracarbonsäure-Bisanhydrid mit o-Phenylendiamin, wobei neben anderen Produkten auch ein Perylentetracarbonsäure-Amidinimid (mit einem 2-Aminophenyl Substituenten am Imidstickstoff) entsteht. Dieses Produkt wird als Küpenfarbstoff eingesetzt. Es wird auch ausgeführt, dass dieses Produkt in organischen Lösungsmitteln nur schlecht löslich ist und eine schwache Fluoreszenz aufweist.

Die US 4,336,383 beschreibt bestimmte 1,1'-Binaphthyl-4,4'5,5'8,8'-hexacarbonsäurederivate, welche ebenso als Küpenfarbstoffe eingesetzt werden. Die Binapthyl-Derivate werden mit Reduktionsmitteln behandelt und anschliessend oxidiert, wobei auf der Textilfaser entsprechende Perylentetracarbonsäure-Amidinimide gebildet werden. Es wird erwähnt, dass diese unlöslichen Amidinimide auch ausserhalb des Farbbades synthetisiert werden können und als Pigmente verwendet werden können.

Y. Nagao, T. Misono, N. Ishikawa und Y. Tanabe beschreiben in Chemistry Letters, **1979**, 151 und in Dyes and Pigments, 1984, **5**, 171 die Herstellung von unsymmetrischen N-Alkyl-N'-aryl-3,4:9,10-perylendicarboximiden durch Kondensation von N-Alkyl-3,4:9,10-perylentetracarbonsäure-monoanyhdrid-monoimiden mit Arylaminen. Beschrieben ist auch die Umsetzung mit o-Phenylendiamin, wobei N-Alkyl-perylentetracarbonsäure-amidinimide mit kurzkettigen, primären Alkylsubstituenten am Imidstickstoff entstehen, sowie die UV Spektren der letztgenannten Verbindungen in konzentrierter Schwefelsäure. Es gibt keinen Hinweis auf eine allfällige Fluoreszenz dieser Verbindungen.

Die US 4,714,666, US 4,968,571 und US 5,019,473 beschreiben die Verwendung bestimmter Perylenpigmente als photoleitfähige Substanzen in der Elektrophotographie. Unter den zu verwendenden Perylenpigmenten werden auch Perylentetracarbonsäureamidinimide mit einem Methyl-, Benzyl- oder Phenylethylsubstituenten am Imidstickstoff erwähnt.

Die erfindungsgemässen Perylenamidinimide der Formel I können durch Umsetzung eines Perylen-3,4:9,10-tetracarbonsäure-monoanhydrids-monoimids der Formel VIII 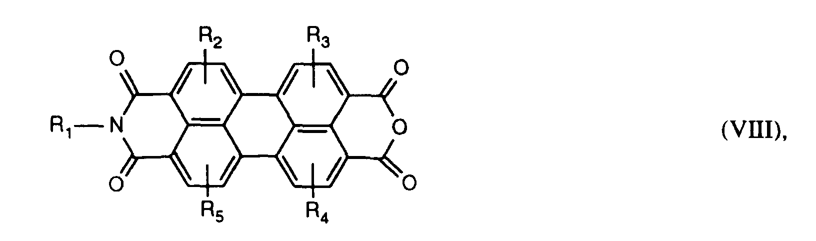 mit einem primären Diamin der Formel IX

H₂N-A-NH₂ (IX),

oder für Verbindungen der Formel I, worin A einen zweiwertigen Rest der Formel III bedeutet, durch Umsetzung des Monoanhydrids-monoimids der Formel VIII mit einem gegebenenfalls substituierten Imidazol hergestellt werden, wobei in den Formeln VIII und IX R₁ und A die oben angegebene Bedeutung haben, mit der Massgabe, dass A nicht für einen Rest der Formel III steht.

Perylen-3,4:9,10-tetracarbonsäure-monoanhydrid-monoimide der Formel VIII sind beispielsweise aus dem Artikel von H. Tröster, Dyes and Pigments, 1983, **4**, 171 bzw. aus dem oben erwähnten Chem. Ber. 1991, **124**, 529 Artikel bekannt, oder sie können auf analoge Weise hergestellt werden.

Als löslichkeitssteigernde Gruppe kann bei der Synthese beispielsweise der sehr wirksame 1-Hexylheptylrest oder ein anderer oben definierter Rest R₁ verwendet werden. Die Synthesen, Aufarbeitungen und spektroskopischen Charakterisierungen der Farbstoffe können damit ganz wesentlich erleichtert werden. So kann z.B. N,N'-Di(1-hexylheptyl)perylen-3,4:9,10-tetracarbonsäurebisimid (**2a**) mit KOH in tert-Butylalkohol partiell verseift werden, so daß nach Ansäuern das Monoanhydrid (**3a**) erhalten wird. Das Aufarbeitungsverfahren kann, wie folgt, erheblich verbessert werden, so daß problemlos mit üblichen Chromatographiesäulen (Durchmesser 4 cm) Farbstoff-Mengen von 30 g oder mehr in einem Lauf hochrein erhalten werden können: Das rohe Reaktionsprodukt der Verseifung, dass das Bisimid (**2a**), Monoanhydrid-Monoimid (**3a**) und Bisanhydrid (**1**) der Perylentetracarbonsäure, sowie Nebenprodukte enthält, wird z.B. mit Chloroform auf Kieselgel aufgezogen. Nebenprodukte und das überschüssige Bisimid werden vollständig aus der Säule mit Chloroform gewaschen - das letztere kann damit auch zurückgewonnen werden. Dann wird dem Elutionsmittel Chloroform 10% Eisessig zugesetzt, und es wird das Monoanhydrid-Monoimid als reine Fraktion erhalten. Das Bisanhydrid und geringe Anteile hochmolekularer Verbindungen verbleiben auf der Säule.

Zur Darstellung eines Monimino-Derivats wird das Anhydrid (**3a**) mit einem primären Diamin der Formel IX, beispeilsweise mit Neopentandiamin, oder bei Verbindungen, worin A einen Rest der Formel III darstellt, mit einem Imidazol kondensiert. Das Einbinden der Imino-Funktion in einen fünf- oder sechsgliedrigen Ring, und im Falle von Neopentandiamin die geminalen Methylgruppen, gewährleisten dabei in erstaunlich hohem Maße eine Beständigkeit der sonst labilen Imino-Funktion gegen hydrolysierende Reagenzien.

Wird bei der Kondensation mit Neopentandiamin die Reaktion wie üblich vorgenommen und aufgearbeitet, dann findet man zum Erstaunen nicht das erwartete Imin, sondern ein Oxydationsprodukt (**5a**), das ebenfalls stark fluoresziert. Das zunächst erwartete Amidin-Imid (**4a**) wird dagegen erhalten, wenn die Kondensation unter Argon-Schutzatmosphäre durchgeführt, und die rohe Reaktionslösung direkt chromatographisch aufgearbeitet wird.

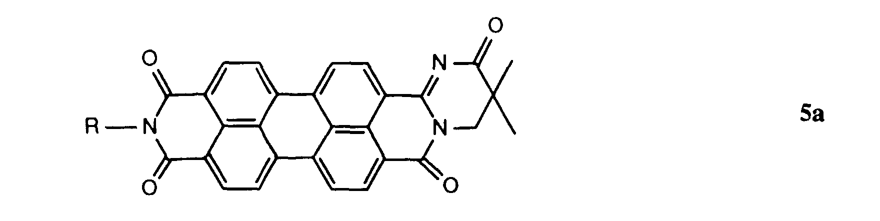

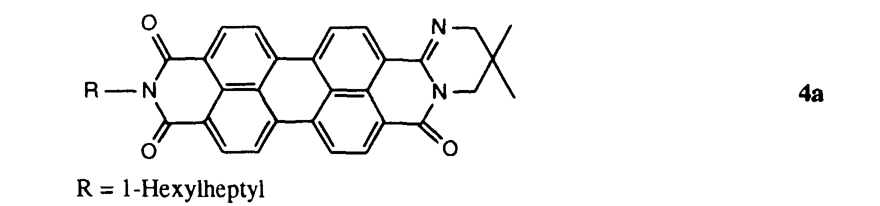

Längerwellig absorbierende Farbstoffe der Formel I werden erhalten, wenn das chromophore System der Amidin-Imid-Farbstoffe weiter ausgedehnt wird. Dies kann beispielsweise durch eine Kondensation des Anhydrid-Imids (**3a**) mit o-Phenylendiamin zum Farbstoff (**6a**) oder durch eine Kondensation mit 1,8-Diaminonaphthalin zum Farbstoff (**7a**) geschehen.

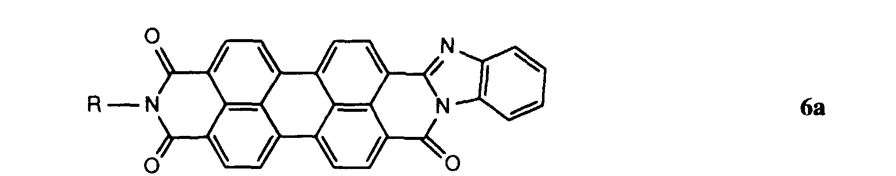

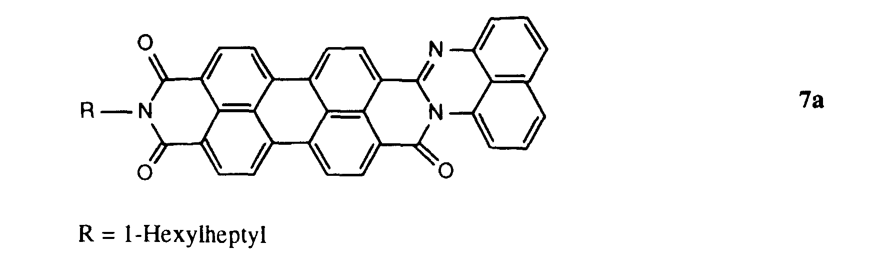

Durch Umsetzung des Anhydrid-Imids (**3a**) mit Imidazol, gegebenenfalls in Gegenwart eines Amins, wie z.B. Trisethylmethylamin oder Ethyldiisopropylamin, kann ein neuer Typ von Amidin-Imid Farbstoff, **8a**, synthetisiert werden, wobei die Imidazolgruppe übertragen wird. Dieses Produkt kann nicht durch eine direkte Kondensation mit dem entsprechenden primären Diamin hergestellt werden, da (Z)-1,2-Diaminoethylen nicht bekannt ist.

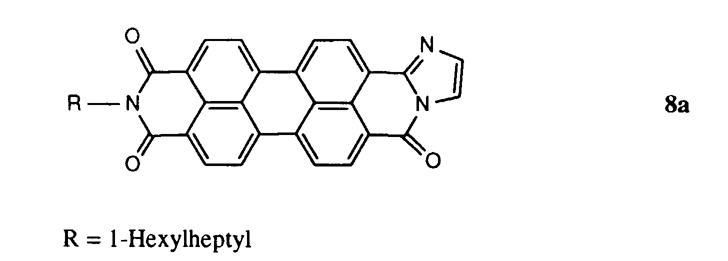

Unter den erfindungsgemässen Verbindungen der Formel I werden solche bevorzugt, worin R₁-CH(R₁₀)₂ bedeutet, und R₁₀ C₄-C₁₈-Alkyl, vorzugsweise C₆-C₁₀-Alkyl ist, oder worin R₁ einen Rest der Formel II mit R gleich tert-Butyl bedeutet. Besonders bevorzugt sind Verbindungen, worin R₁ 2,5-Di-tert-butylphenyl oder -CH(R₁₀)₂ bedeutet und R₁₀ ein geradkettiger Rest, insbesondere n-Hexyl, n-Heptyl, n-Octyl, n-Nonyl oder n-Decyl, ist.

Bei der Herstellung der Verbindungen der Formel I wird das Monoanhydrid-Monoimid der Formel VIII vorzugsweise mit aliphatischen, cycloaliphatischen, aromatischen oder heteroaromatischen Diaminen umgesetzt, welche ein Amidin-Imid-Produkt mit einem fünf- oder sechsgliedrigen Amidinring ergeben. Beispiele bevorzugter Diamine sind 1,2-Cyclopentandiamin, 1,2-Cyclohexandiamin, o-Phenylendiamin, 2,3- oder 1,8-Diaminonaphthalin, 2,3- oder 3,4-Diaminopyridin, 9,10-Diaminophenanthren, oder Diamine, von denen ein zweiwertiger Rest der Formel IV oder V abgeleitet wird, sowie gegebenenfalls substituierte Imidazole, wie z.B. Imidazol oder 2-Aminobenzimidazol. Besonders bevorzugte Diamine sind o-Phenylendiamin, 1,8-Diaminonaphthalin, Neopentandiamin oder Imidazol.

Bevorzugte im Perylenkern substituierte Perylenamidinimide der Formel I sind solche Verbindungen, worin die Substituenten R₂ bis R₅ unabhängig voneinander H, Halogen, Nitro, -OR₈, -NR₈R₉, -NR₈COR₉, -SO₃R₈ oder -SO₂NR₈R₉ bedeuten und R₈ und R₉ unabhängig voneinander vorzugsweise Methyl, Phenyl oder 4-Tolyl sind. Die kernsubstituierten Verbindungen können analog den nichtsbustituierten Verbindungen nach allgemein bekannten Methoden hergestellt werden. Die Einführung der Substituenten erfolgt dabei vorzugsweise auf der Stufe des Perylenbisimid-Ausgangsmaterials, d.h. vor der Herstellung des Monoanhydrid-Monoimids der Formel VIII. Am meisten bevorzugt sind allerdings Perylenamidinimide der Formel I, worin R₂ bis R₅ H oder jeweils den gleichen Substituenten darstellen, und insbesondere solche, worin mindestens zwei, vorzugsweise alle Reste R₂ bis R₅ H bedeuten.

Wie bereits erwähnt, zeichnen sich die erfindugnsgemässen Amidinimid-Farbstoffe durch ihre ausgeprägte Lichtechtheit aus, so daß sie insbesondere für Fluoreszenzanwendungen von Interesse sind. Gegenstand der Erfindung ist somit auch die Verwendung dieser Verbindungen als Fluoreszenzfarbstoffe, insbesondere in Chemilumineszenzsystemen, in optischen Lichtsammelsystemen, in Fluoreszenz-Solarkollektoren (siehe z.B. H. Langhals, Nachr. Chem. Tech. Lab., 1980, **28**, 716), in Fluoreszenez-aktivierten Displays (siehe W. Greubel, G. Baur, Elektronik, 1977, **26**, 6) oder in der "Single-Molecule-Spectroscopy" (siehe W.E. Moerner, T. Basché, Angew. Chem. 1993, 105, 537), zum Beispiel zum Aufbau von optischen Speichern.

Ein weiterer Gegenstand ist auch deren Verwendung als photoleitfähige Substanzen in der Elektrophotographie.

Die Perylenamidinimide der Formel I eignen sich auch vorzüglich für die Massefärbung von Kunststoffen. Sie können vorteilhaft zum Massefärben von verschiedenen Polymeren, wie z.B. Polyolefine, Polyvinylchlorid, Fluorpolymerisate, wie z.B. Polyfluorethylen, Polytrifluorchlorethylen oder Tetrafluorethylen/Hexafluorpropylen-Mischpolymerisat, Silikonharze, insbesondere aber von Ingenieurwerkstoffen (Engineering Plastics), wie z.B. Polycarbonate, Polyacrylate, Polymethacrylate, Polystyrol, ABS, Polyester, insbesondere Polyalkylenterephthalate, wie Polybutylenterephthalat (PBT) oder Polyethylenterephthalat (PET), Polyamide, Polyetherketone, Polyurethane, einzeln oder in Mischungen, verwendet werden. Zweckmässig werden sie in einer Konzentration von 0.01 bis 10, vorzugsweise 0.01-5 Gew.%, bezogen auf das Polymere, eingesetzt.

Als Beispiele für Polyolefine, die mit den erfindungsgemässen Verbindungen der Formel I gefärbt werden können, seien Polyethylen hoher und niederer Dichte (HD-PE, LD-PE und LLD-PE), Polyisobutylen und insbesondere Polypropylen, sowie Copolymere von Polyolefinen mit z.B. Polyethern, Polyetherketonen oder Polyurethanen, erwähnt. Bevorzugt ist Polypropylen.

Die Färbung erfolgt nach den üblichen Verfahren, beispielsweise durch Mischen einer Verbindung der Formel I oder eines Gemisches solcher Verbindungen mit dem Kunststoffgranulat oder -pulver, ohne es vorher in ein Präparat einarbeiten zu müssen, und Extrudieren der Mischung zu Fasern, Folien oder Granulaten. Letztere können dann beispielsweise im Spritzgussverfahren zu Gegenständen verformt werden.

Die erhaltenen rot fluoreszierenden Ausfärbungen weisen hohe Reinheit und hohe Sättigung auf und zeichnen sich durch gute Transparenz sowie durch gute Beständigkeit, insbesondere gegen Licht aus. Ein weiterer Gegenstand der Erfindung ist somit auch in der Masse gefärbtes hochmolekulares organisches Material enthaltend als Farbmittel ein Perylenamidinimid der Formel I.

Die nachfolgenden Beispiele erläutern die Erfindung.

### Beispiel 1: N-(1-Hexylheptyl)-perylen-3,4:9,10-tetracarbonsäure-3,4-anhydrid-9,10-imid (3a)

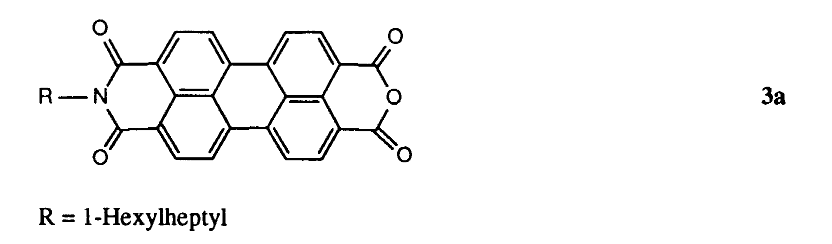

8.00 g (10.6 mmol) N,N'-Di-(1-hexylheptyl)-perylen-3,4:9,10-bisdicarboximid werden in 50 ml tert-Butylalkohol eingetragen und die Lösung zum Sieden erhitzt. In die Lösung werden 2.38 g (36.0 mmol) pulverisiertes 85 proz. KOH unter Rühren eingetragen. Nach 10 min ist dünnschichtchromatographisch kein Edukt mehr nachzuweisen. Die Verseifung wird durch Zugabe von 100 ml Eisessig gefolgt von 100 ml 2 N HCl abgebrochen. Das ausgefallene rote Farbstoffrohprodukt wird durch Absaugen der Lösung über eine D-4 Fritte isoliert und mehrfach mit dest. Wasser gewaschen. Zur Abtrennung des mitentstandenen Perylentetracarbonsäure-3,4:9,10-bis(dicarboxanhydrid) wird das Rohprodukt 2 mal mit jeweils 300 ml 10 proz. Kaliumcarbonat-Lösung aufgekocht und über eine D-4 Fritte abgesaugt. Nach 8 h Trocknen bei 100°C erhält man 4.5 g (74%) Rohprodukt, das durch Säulenchromatographie an Kieselgel mit Chloroform/Eisessig (10:1) als Laufmittel von geringen Resten des Eduktes und weiteren Verunreinigungen befreit wird. Die dabei erhaltene Farbstofffraktion wird einrotiert und nach Zugabe von dest. Wasser über eine D-4 Fritte abgesaugt, gewaschen und getrocknet. Ausb. 3.8 g (63 %), Schmp. 330°C, R_{f} (Kieselgel/CHCl₃)= 0.52. UV (CHCl₃): λₘₐₓ(ε)= 522.1 nm (81750), 486 (52760), 456.4 (23740). Fluoreszenz (CHCl₃) λₘₐₓ= 528 nm, 570.

| | | | | |
|---|---|---|---|---|
| C₃₇H₃₅NO₅ (573.7) | Ber. | C 77.46 | H 6.15 | N 2.44 |
| | Gef. | C 77.46 | H 5.96 | N 2.49 |

### Beispiel 2: N-(1-Heptyloctyl)-perylen-3,4:9,10-tetracarbonsäure-3,4-anhydrid9,10-imid (3b)

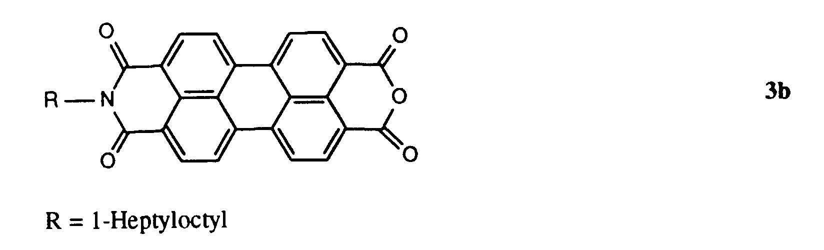

4.05 g (5.00 mmol) N,N'-Di(1-heptyloctyl)-perylen-3,4:9,10-bis(dicarboximid) werden in 100 ml tert-Butylalkohol gelöst, und dann unter Rühren mit 1.68 g (25.4 mmol) 85 proz. pulverisiertem Kaliumhydroxid versetzt. Die Reaktionsmischung wird zum Sieden erhitzt und das Fortschreiten der Verseifung dünnschichtchromatographisch verfolgt (Kieselgel/CHCl₃). Die Reaktion wird nach 12 min durch Zugabe von 120 ml Eisessig unter Eiskühlung abgebrochen. Das Edukt ist dünnschichtchromatographisch nicht mehr nachzuweisen. Nach Zugabe von 50 ml 2 N HCl wird mit einer D-4 Fritte abgesaugt und der rotbraune Feststoff mit dest. Wasser neutralgewaschen. Zur Abtrennung von mitentstandenem Perylentetracarbonsäurebisanhydrid wird das Rohprodukt zweimal mit je 200 ml 10 proz. Kaliumcarbonat-Lösung aufgekocht und dann mit einer D-4 Fritte abgesaugt. Das erhaltene Rohprodukt wird 8 h bei 100°C getrocknet. Ausb. 1.7 g (56%). Das Rohprodukt wird durch Säulenchromatographie (80 x 4cm) an Kieselgel mit Chloroform/Eisessig 10:1 als Laufmittel gereinigt. Spuren von Verunreinigungen können durch erneute Säulenchromatographie an Kieselgel mit Chloroform/Triethylamin 10:1 als Laufmittel entfernt werden. Die isolierte Farbstofffraktion wird mit Eisessig versetzt, einrotiert und der ausfallende Farbstoff mit Wasser gewaschen und 8 h im Ölpumpenvakuum bei 80°C getrocknet. Ausb. 1.44 g (48%), Schmp. 316°C, R_{f} (Kieselgel/CHCl₃)= 0.44, R_{f} (Kieselgel/CHCl₃/Eisessig 10:1)= 0.85. UV (CHCl₃): λₘₐₓ(ε)=455 nm (17350), 485 (46260), 522 (70380). Fluoreszenz (CHCl₃):λₘₐₓ=528 nm, 570.

| | | | | |
|---|---|---|---|---|
| C₃₉H₃₉NO₅ (601.7) | Ber. | C 77.84 | H 6.53 | N 2.33 |
| | Gef. | C 78.04 | H 6.64 | N 2.54 |

### Beispiel 3: N-(1-Octylnonyl)-perylen-3,4:9,10-tetracarbonsäure-3,4-anhydrid-9,10-imid (3c)

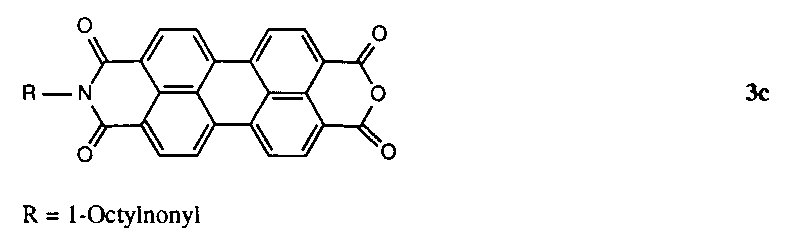

8.67 g (9.99 mmol) N,N'-Di-(1-octylnonyl)perylen-3,4:9,10-bis(dicarboximid) werden in 100 ml tert-Butylalkohol unter Erwärmen gelöst. In die zum Sieden gebrachte Lösung werden 2.24 g (33.9 mmol) pulverisiertes 85 proz. KOH eingetragen, und die Reaktions-Mischung 12 min unter Rühren am Sieden gehalten. Die Verseifung wird nun durch Zugabe von 100 ml Eisessig und 100 ml 2 N HCl unter Eiskühlung abgebrochen. Der ausgefallene rote Niederschlag wird nach Zugabe von 200 ml dest. Wasser über eine D-4 Fritte abgesaugt, neutralgewaschen und zweimal mit je 200 ml 10 proz. Kaliumcarbonat-Lösung aufgekocht und abgesaugt. Der Niederschlag wird 8 h bei 100°C getrocknet. Ausb. 4.15 g (66 %) Rohprodukt. Zur weiteren Reinigung wird das Rohprodukt mit CHCl₃/Eisessig 10:1 an Kieselgel chromatographiert (Säule 80 x 4 cm). Zur Elementaranalysenreinheit wird der dabei erhaltene Farbstoff erneut mit CHCl₃/Triethylamin 10:1 als Laufmittel chromatographiert. Ausb. 3.68 g (58%), Schmp. 312°C, R_{f} (Kieselgel/CHCl₃)= 0.36, R_{f} (Kieselgel/CHCl₃/Eisessig 10:1)= 0.66. UV (CHCl₃): λₘₐₓ(ε)= 456 nm (18670), 485 (46850), 521 (75710). Fluoreszenz (CHCl₃): λₘₐₓ= 528 nm, 570.

| | | | | |
|---|---|---|---|---|
| C₄₁H₄₃NO₅ (629.7) | Ber. | C 78.18 | H 6.88 | N 2.22 |
| | Gef. | C 77.84 | H 6.84 | N 2.24 |

### Beispiel 4: N-(1-Nonyldecyl)-perylen-3,4:9,10-tetra carbonsäure-3,4-anhydrid9,10-imid (3d)

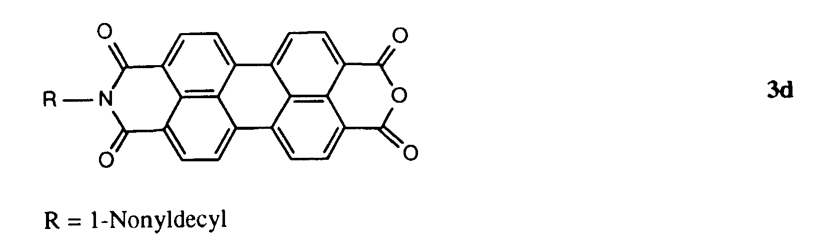

923 mg (1.00 mmol) N,N'-(1-Nonyldecyl)-perylen-3,4:9,10-tetracarbonsäure-bis(dicarboximid) werden in 50 ml tert-Butylalkohol unter Erwärmen gelöst und unter Rühren mit 225 mg (3.41 mmol) 85 proz. pulverisiertem KOH versetzt. Die Lösung wird zum Sieden erhitzt und der Verlauf der Verseifung dünnschichtchromatographisch verfolgt. Nach 13 min kann kein Edukt mehr nachgewiesen werden. Die Reaktion wird durch Zugabe von 50 ml Eisessig und 50 ml 2 N HCl unter Eiskühlung abgebrochen. Der rotbraune Niederschlag wird über eine D-4 Fritte abgesaugt, mit dest. Wasser neutralgewaschen und 8 h bei 100°C getrocknet. Ausb. 620 mg (94.1%) Rohprodukt. Die weitere Reinigung erfolgt analog zu N-(1-Heptyloctyl)-perylen-3,4:9,10-tetracarbonsäure-3,4-anhydrid-9,10-carboximid. Ausb. 330 mg (50.1%), Schmp. 308°C, R_{f} (Kieselgel/CHCl₃)= 0.77, R_{f} (Kieselgel/CHCl₃/Eisessig 10: 1)= 0.85. UV (CHCl₃): λₘₐₓ(∈)= 455 nm (20690), 486 (48920), 522 (77990). Fluoreszenz(CHCl₃): λₘₐₓ= 528 nm, 870.

| | | | | |
|---|---|---|---|---|
| C₄₃H₄₇NO₅ (657.8) | Ber. | C 78.51 | H 7.20 | N 2.13 |
| | Gef. | C 78.53 | H 7.14 | N 2.24 |

### Beispiel 5: N-(1-Decylundecyl)-perylen-3,4:9,10-tetracarbonsäure-3,4-anhydrid-9,10-imid (3e)

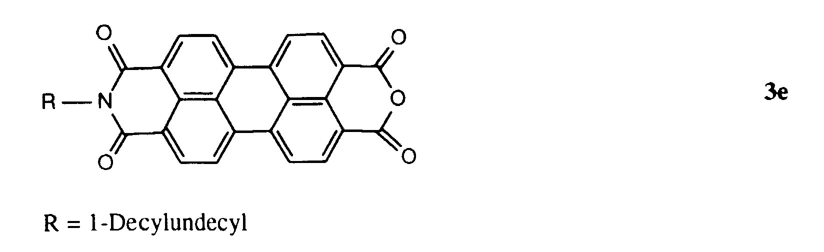

4.98 g (5.00 mmol) N,N'-Di-(1-decylundecyl)-perylen-3,4:9,10-tetracarbonsäurebis(dicarboximid) werden in 100 ml tert-Butylalkohol gelöst und unter Rühren mit 1.12 g (17.0 mmol) pulverisiertem 85 proz. KOH versetzt. Die Lösung wird zum Sieden erhitzt und der Verlauf der Verseifung dünnschichtchromatographisch verfolgt. Nach 12 min wird die Reaktion durch Zugabe von 150 ml Eisessig, unter Eiskühlung, abgebrochen. Die weitere Reinigung erfolgt analog zu N-(1-Heptyloctyl)-perylen-3,4:9,10-tetracarbonsäure-3,4-anhydrid-9,10-imid. Ausb. 1.95 g (57%), Schmp. 302°C, R_{f} (Kieselgel/CHCl₃)= 0.80, R_{f} (Kieselgel/CHCl₃/Eisessig 10:1)= 0.85. UV (CHCl₃): λₘₐₓ(ε)= 455 nm (18470), 486 (47840), 522 (77690). Fluoreszenz (CHCl₃): λₘₐₓ= 528 nm, 870.

| | | | | |
|---|---|---|---|---|
| C₄₅H₅₁NO₅ (685.8) | Ber. | C 78.81 | H 7.49 | N 2.04 |
| | Gef. | C 78.76 | H 7.46 | N 2.18 |

### Beispiel 6: 2-(Hexylheptyl)-3,3-dimethylpyrimido[2,1-a]anthra[2,1,9-def:6,5,10-d'e'f']-diisochinolin-2,6,11,13(3H,4H,12H)-tetron (5a)

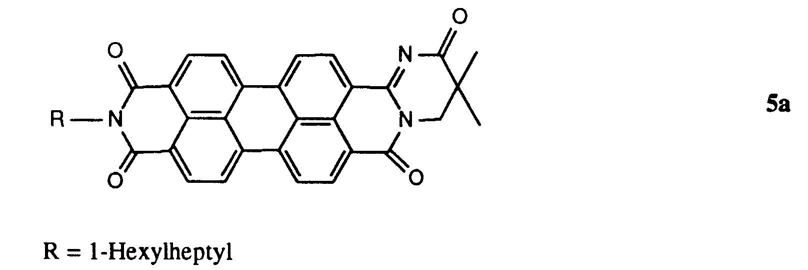

2.00 g (3.49 mmol) N-(1-Hexylheptyl)-3,4:9,10-tetracarbonsäure-3,4-anhydrid-9,10-imid werden mit 10.4 g (102 mmol) Neopentandiamin und 10.0 g Imidazol unter Ausschluß von Kohlendioxid (KOH) in einer mit Argon gespülten Apparatur 90 min auf 180°C erhitzt. Nach kurzer Zeit tritt dabei ein Farbumschlag nach Schwarzviolett auf. Die Reaktionsmischung wird nach dem Erkalten mit 100 ml Ethanol aus dem Reaktionsgefäß gespült und dann mit 100 ml 2N Salzsäure versetzt. Man läßt mindestens 1 h bei Raumtemperatur rühren und saugt dann den feinen, violettbraunen, fast schwarzen Niederschlag ab, der 12 h bei 130°C an der Luft getrocknet wird. Ausb. 2.14 g (95%). Dünnschichtchromatographisch (Kieselgel, Chloroform/Eisessig 10:1) werden außer dem Edukt (R_{f}= 0.89) und einer gelborange fluoreszierenden Verbindung noch drei rotorange fluoreszierende Produkte mit den R_{f}-Werten 0.00, 0.25 und 0.83 gefunden. Säulenchromatographisch (Kieselgel, Chloroform/Eisessig 10:1) wird das Produkt mit dem R_{f}-Wert von 0.83 isoliert und nochmals mit Chloroform/1-Butanol 40:1 chromatographiert. Das Eluat wird durch eine D5-Glasfritte filtriert und nach Abdampfen des Lösungsmittels bei 130°C getrocknet. Ausb. 1.36 g (60%), Schmp. >360°C, R_{f} (Kieselgel, CHCl₃/Eisessig 10:1)= 0.83.

| | | | | | |
|---|---|---|---|---|---|
| C₄₂H₄₃N₃O₄ (653.8) | Ber. | C 77.16 | H 6.63 | N 6.43 | O 9.79 |
| | Gef. | C 77.41 | H 6.67 | N6.28 | O 9.75 |

### Beispiel 7: 12-(Hexylheptyl)-3,3-dimethylpyrimido[2,1-a]anthra[2,1,9-def:6,5,10-d'e'f']-diisochinolin-6,11,13(2H,3H,4H,12H)-trion (4a)

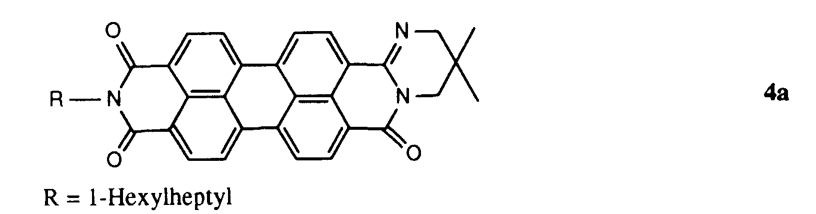

2.00 g (3.49 mmol) N-(1-Hexylheptyl)-3,4:9,10-tetracarbonsäure-3,4-anhydrid-9,10-imid werden mit 10.4 g (102 mmol) Neopentandiamin und 10.0 g Imidazol analog wie bei **5a** unter Ausschluß von Luft (Argon-Schutzatmosphäre) 90 min auf 180°C erhitzt. Nach kurzer Zeit tritt dabei ein Farbumschlag nach Schwarzviolett auf. Eine dann sofort entnommene Probe ergibt die gleichen Produkte, wie bei **5a**, aber in einem anderen Verhältnis. Ohne Aufarbeitung mit Ethanol/HCl wird der rohe Ansatz mit Chloroform/Eisessig 10:1 über eine Säule mit Kieselgel chromatographiert. Die ersten drei Fraktionen werden verworfen und das rote Produkt mit einem R_{f}-Wert von 0.25 isoliert und bei 130°C an der Luft getrocknet. Ausb. 220 mg (10%), Schmp. >310°C, R_{f} (Kieselgel, CHCl₃/Eisessig 10:1)= 0.25. UV (CHCl₃): λₘₐₓ= 538 nm, 501, 469. Fluoreszenz (CHCl₃): λₘₐₓ= 559 nm, 591. MS (70 eV): m/z (%) = 639 (46) [M⁺].

### Beispiel 8: 13-(1-Hexylheptyl)benz[3,4]imidazolo[2,1-a]anthra[2,1,9-def:6,5,10-d'e'f']di-isochinolin-7,12,14,13H-trion (6a)

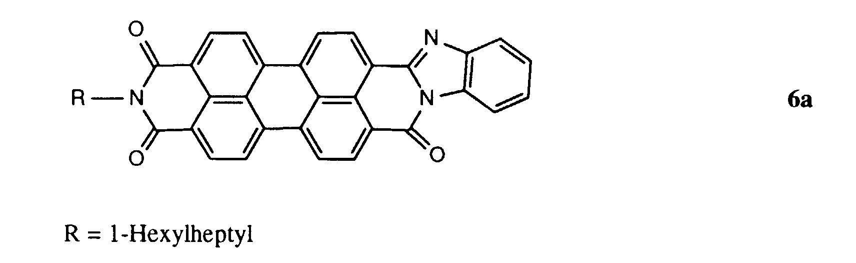

1.00 g (1.74mmol) N-(1-Hexylheptyl)-3,4:9,10-tetracarbonsäure-3,4-anhydrid-9,10-imid, 5.00g (46.2 mmol) 1,2-Diaminobenzol und 5.00 g Imidazol werden unter Kohlendioxid-Ausschluß in einer mit Argon gespülten Apparatur 2 h auf 180°C erhitzt. Das feste, schwarzbraune Reaktionsprodukt wird mit 150 ml Ethanol aufgeschlämmt und die dabei entstandene schwarzbraune Suspension mit 100 ml 2 N Salzsäure versetzt und 1 h bei Raumtemperatur gerührt. Dann wird abgesaugt und der violettbraune Feststoff 16 h bei 130°C getrocknet. Ausb. 1.07 g (88%). Das Rohprodukt enthält neben dem pink fluoreszierenden Reaktionsprodukt (mit einem R_{f}-Wert von 0.88 (Chloroform/Kieselgel) das Edukt (R_{f}= 0.89) und ein braunes, nicht fluoreszierendes Nebenprodukt mit einem R_{f}-Wert von 0.00. Zur Isolation des Hauptprodukts wird an Kieselgel mit Chlorform/Eisessig 10:1 und dann noch einmal mit Chloroform/Butanol 40:1 chromatographiert. Die Haupfraktion der zweiten Chromatographie wird eingeengt, und der Farbstoff kristallisiert aus Butanol in glänzenden, rotvioletten Nädelchen aus. Die Kristalle werden abgesaugt, mehrmals mit dest. Wasser gewaschen und bei 130°C getrocknet. Ausb. 840 mg (74%), Schmp. >360°C, R_{f} (CHCl₃/Eisessig 10:1)= 0.88. UV (CHCL₃): λₘₐₓ (ε)= 571 nm (57360), 535 (50820), 504 (26760). Fluoreszenz (CHCl₃): λₘₐₓ= 602 nm.

| | | | | |
|---|---|---|---|---|
| C₄₃H₃₉N₃O₃ (645.8) | Ber. | C 79.97 | H 6.09 | N 6.57 |
| | Gef. | C 79.66 | H 6.02 | N 6.53 |

### Beispiel 9: 15-(1-Hexylheptyl)perimidino[2,1-a]anthra[2,1,9-def:6,5,10-d'e'f']diisochinolin-9,14,16(15H)-trion (7a)

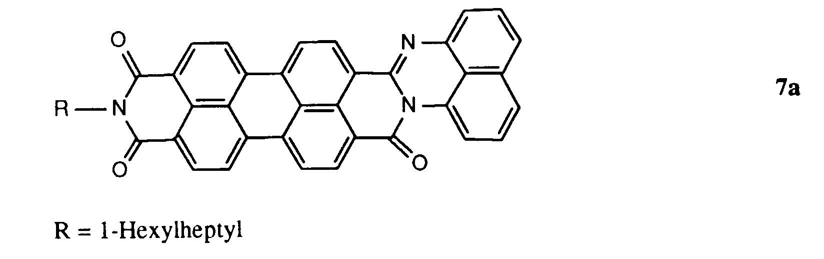

1.00 g (1.74 mmol) N-(1-Hexylheptyl)-3,4:9,10-perylentetracarbonsäure-3,4-anhydrid-9,10-imid (**3a**) werden in einer mit Argon gespülten Apparatur mit 10.0 g (63.2 mmol) 1,8-Naphthalindiamin, 30 ml frisch destilliertem Chinolin und 330 mg Zinkacetat auf 160°C erhitzt. Nach 30 min schäumt die Reaktionsmischung heftig auf und bildet an der Oberfläche einen zähen Überzug. Nach weiteren 30 min hat sich die Mischung nach Tiefviolett verfärbt. Die Reaktionsmischung wird eine weitere Stunde auf 160°C erhitzt, und nach dem Abkühlen wird der zähe Brei in 300 ml Ethanol suspendiert und 16 h bei Raumtemperatur gerührt. Der schwarze Niederschlag wird abgesaugt, solange mit Ethanol gewaschen, bis der Chinolin-Geruch verschwunden ist und dann bei 130°C getrocknet. Ausb. 1.15 g (94%). Im Dünnschichtchromatogramm dieses Produkts (Kieselgel, Chloroform/Eisessig 10:1) erkennt man neben dem Ausgangsmaterial eine blaue, verschmierte Zone. Zur Abtrennung des Edukts verwendet man seine gute Löslichkeit in Chloroform/Eisessig-Gemischen, während das Reaktionsprodukt mit zunehmendem Eisessig-Anteil schwerer löslich wird. Das Rohprodukt wird mit jeweils 11 Chloroform/Eisessig 10:1 digeriert und dann abfiltriert. Aus der Lösung werden 800 ml Chloroform abdestilliert, und der Rest wird 16 h bei Raumtemperatur stehengelassen. Der dabei in winzigen Nädelchen auskristallisierende, violettblaue Farbstoff wird über eine D4-Glasfritte abgesaugt, mehrfach mit dest. Wasser gewaschen und bei 130°C getrocknet. Diese Prozedur wird so lange wiederholt, bis das ganze Rohprodukt gelöst ist. Ausb. 320 mg (26%), Schmp > 360°C, R_{f} (Al₂O₃/CHCl₃)= 0.95. UV (CHCl₃): λₘₐₓ= 604 nm, 542, 507.

| | | | | |
|---|---|---|---|---|
| C₄₇H₄₁N₃O₃ (695.9) | Ber. | C 81.13 | H 5.94 | N 6.04 |
| | Gef. | C 79.90 | H 5.85 | N 6.03 |

### Beispiel 10: 11-(11-(1-Hexylheptyl)imidazolo[2,1-a]anthra[2,1,9-def:6,5,10-d'e'f']diisochinolin-5,10,12(11H)-trion (8a)

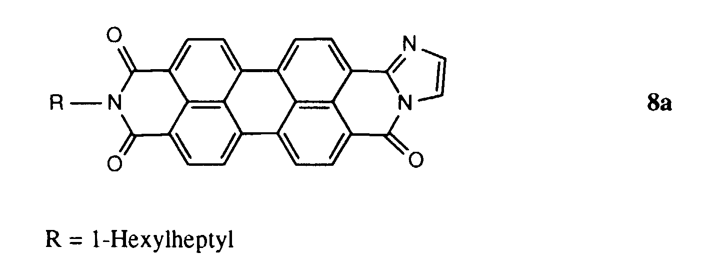

0.5 g N-(1-Hexylheptyl)-perylen-3,4,9,10-tetracarbonsäure-3,4-anhydrid-9,10-imid werden mit 0.77 g Trisethylmethylamin (frisch destilliert) und 0.8 g Imidazol gut gemischt und in ein Autoklavenrohr gefüllt. Das Reaktionsgemisch wird im Autoklaven während 4 h auf 170°C erhitzt. Nachdem man den Autoklaven über Nacht auf Raumtemperatur abkühlen lässt, wird er geöffnet, und das Produktgemisch wird mit 200 ml siedendem Ethanol herausgelöst. Durch Zusatz von 400 ml 2 N HCl wird das Produkt gefällt. Zur Vervollständigung der Ausfällung wird noch 1 h bei Raumtemperatur gerührt, der rotviolette Niederschlag wird dann über eine D-4 Glasfilterfritte abgesaugt und anschliessend 8 h bei 110°C getrocknet. Man Erhält 0.48 g rotviolettes Pulver als Rohprodukt. Zur ersten Auftrennung werden 0.2 g des Rohproduktes in wenig Chloroform gelöst und säulenchromatographisch an Aluminiumoxid (neutral) aufgetrennt (Chloroform/Eisessig 10:1). Neben anderen nicht identifizierten Produkten und nicht umgesetztem Monoanhydrid-Monoimid isoliert man ein stark fluoreszierendes Hauptprodukt, das an Kieselgel mit Chloroform/Eisessig (10:1) hochgereinigt wird. Ausbeute 0.12 g (24%) schwarzviolettes Pulver. R_{f}(Kieselgel/CHCl₃)=0.01, R_{f}(CHCl₃/Eisessig 10:1)=0.47, UV (CHCl₃:λₘₐₓ=487 nm, 520, 560, MS (70eV):m/z(%)=595.3(15)[M⁺]. Anhand dieser Daten sowie der IR, ¹H-NMR und ¹³C-NMR lässt sich das Produkt als Verbindung 8a identifizieren.

| | | | | |
|---|---|---|---|---|
| C₃₉H₃₇N₃O₃ (595.7) | Ber. | C 78.63 | H 6.26 | N 7.05 |
| | Gef. | C 78.71 | H 6.38 | N 6.86 |

### Beispiel 11: 15-(1-Hexylheptyl)naphtho[3,2-d]-imidazolo[3,2-a]anthra[2,1,9-def:6,5,10-d'e'f']diisochinolin-9,14,16(15H)-trion

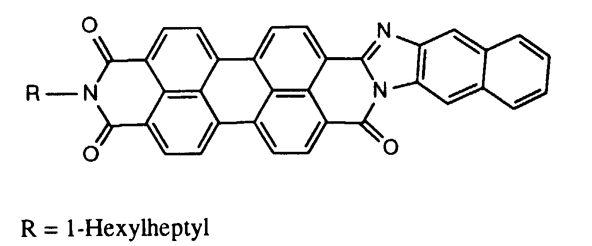

1.00 g (1.74 mmol) N-(1-Hexylheptyl)-3,4:9,10-perylentetracarbonsäure-3,4-anhydrid-9,10-imid (**3a**) und 0.66 g (4.20 mmol) 2,3-Diaminonaphthalin werden in 10.0 g Imidazol 1h bei 160°C gerührt. Der noch flüssige Rückstand wird mit 200 ml Ethanol aus dem Reaktionsgefäss geschwemmt und zur Entfernung von überschüssigem Amin mit 100 ml 2N Salzsäure versetzt. Es wird 1h bei Raumtemperatur gerührt, der ausgefallene violett-schwarze Niederschlag wird über eine D4-Glasfritte abgesaugt und getrocknet. Die Reinigung erfolgt säulenchromatographisch an Kieselgel mit einem Gemisch aus Chloroform/Eisessig (10:1) als Laufmittel, wobei das Produkt als violette Bande eluiert. Nach Entfernen des Lösungsmittels wird der Farbstoff erneut unter den gleichen Bedingungen chromatographiert, das Lösungsmittel wird am Rotationsverdampfer entfernt und der Rückstand wird im Vakuum getrockent. Ausbeute 0.67 g (55%), Schmp > 300°C, R_{f} (CHCl₃/Eisessig 10:1)= 0.57. UV (CHCl₃): λₘₐₓ=567 nm, 538, 383, 363; Fluoreszenz (CHCl₃): λₘₐₓ= 652 nm,

| | | | | |
|---|---|---|---|---|
| C₄₇H₄₁N₃O₃ (695.9) | Ber. | C 81.13 | H 5.94 | N 6.04 |
| | Gef. | C 80.92 | H 5.98 | N 6.14 |

### Beispiel 12: 17-(1-Hexylheptyl)anthra-9,10-dion[2,1-d]-imidazolo[3,2-a]anthra[2,1,9-def:6,5,10-d'e'f']diisochinolin--11,16,18(17H)-trion

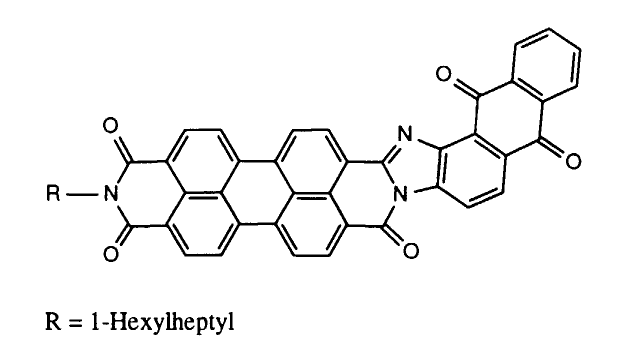

0.20 g (0.34 mmol) N-(1-Hexylheptyl)-3,4:9,10-perylentetracarbonsäure-3,4-anhydrid-9,10-imid (**3a**) und 0.19 g (0.82 mmol) 1,2-Diaminoanthrachinon werden in 2.0 g Imidazol 1h bei 160°C gerührt. Der noch flüssige Rückstand wird mit 80 ml Ethanol aus dem Reaktionsgefäss geschwemmt und zur Entfernung von überschüssigem Amin mit 40 ml 2N Salzsäure versetzt. Es wird 1h bei Raumtemperatur gerührt, der ausgefallene Niederschlag wird über eine D4-Glasfritte abgesaugt und getrocknet. Die Reinigung erfolgt säulenchromatographisch an Kieselgel mit einem Gemisch aus Chloroform/Eisessig (10:1) als Laufmittel, wobei das Produkt als violette Bande eluiert. Nach Entfernen des Lösungsmittels wird der Farbstoff zweimal an Kieselgel mit einem Gemisch aus Chloroform/n-Butanol (40:1) chromatographiert. Schliesslich wird er aus Toluol extraktiv umkristallisiert. Ausbeute 0.13 g (51%); Schmp > 300°C; R_{f} (CHCl₃/n-Butanol 40:1)=0.43; UV (CHCl₃): λₘₐₓ= 574 nm, 535, 500; MS(70eV): m/z(%)= 775(6), 758(3), 593(71), 565(8), 281(10), 207(100), 191(12), 133(10).

### Beispiel 13: 18-(1-Hexylheptyl)phenanthro[9,10-d]-imidazolo[3,2-a]anthra[2,1,9-def:6,5,10-d'e'f']diisochinolin--12,17,19(18H)-trion

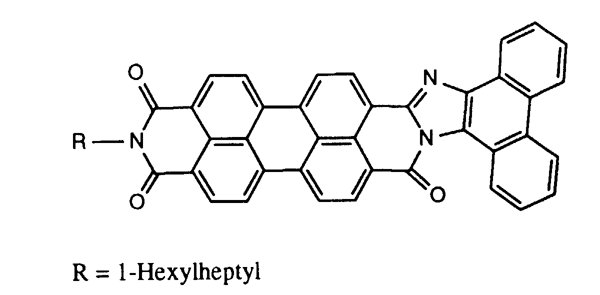

0.20 g (0.34 mmol) N-(1-Hexylheptyl)-3,4:9,10-perylentetracarbonsäure-3,4-anhydrid-9,10-imid (**3a**) und 0.14 g (0.82 mmol) 9,10-Diaminophenanthren werden in 2.0 g Imidazol 1h bei 160°C gerührt. Der noch flüssige Rückstand wird mit 80 ml Ethanol aus dem Reaktionsgefäss geschwemmt und zur Entfernung von überschüssigem Amin mit 40 ml 2N Salzsäure versetzt. Es wird 1h bei Raumtemperatur gerührt, der ausgefallene Niederschlag wird über eine D4-Glasfritte abgesaugt und getrocknet. Die Reinigung erfolgt säulenchromatographisch an Kieselgel mit einem Gemisch aus Chloroform/n-Butanol (40:1) als Laufmittel, wobei sich die Erkennung der einzelnen Banden als äusserst schwierig erweist. Nachdem der Farbstoff dreimal unter diesen Bedingungen chromatographiert wurde, zeigt ein Dünnschichtchromatogramm das fluoreszierende Produkt und eine nicht fluoreszierende Verbindung am Start, die auch durch erneutes Chromatographieren unter den gleichen Bedingungen nicht entfernt werden kann. Ausbeute 0.11 g (44%); Schmp > 300°C; R_{f} (CHCl₃/n-Butanol 40:1)=0.57; UV (CHCl₃): λₘₐₓ= 583 nm, 523, 359; MS(70eV): m/z(%)= 745(50), 728(6), 563(100), 518(13), 493(7), 259(7).

## Patentansprüche

1. Perylenamidinimide der Formel I 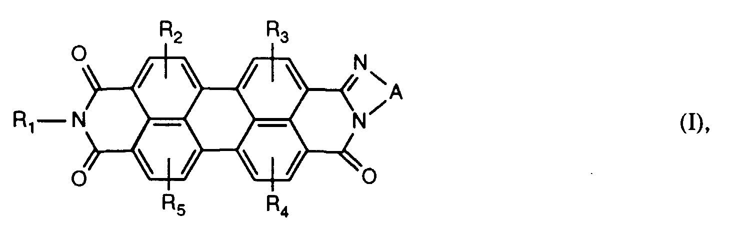 worin R₁ einen sekundären C₇-C₄₁-Alkylrest oder einen Rest der Formel II bedeutet 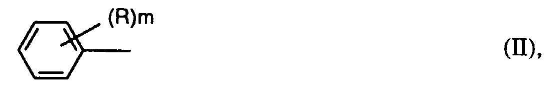 worin R ein verzweigter C₃-C₈-Alkylrest ist und m 1, 2 oder 3 bedeutet;
A für C₅-C₇-Cycloalkylen, Phenylen, Naphthylen, Pyridylen, einen höher kondensierten aromatischen carbocyclischen oder heterocyclischen Rest oder einen zweiwertigen Rest der Formel III, IV oder V 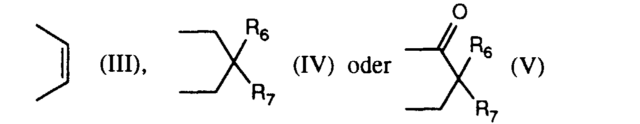 steht, wobei sowohl R₁ als auch A durch Halogen, Alkyl, Cyano oder Nitro substituiert sein können;
R₂ bis R₅ unabhängig voneinander H, Alkyl, Aryl, Heteroaryl, Halogen, Cyano, Nitro, -OR₈, -COR₈, -COOR₈, -OCOR₈, -CONR₈R₉, -OCONR₈R₉, -NR₈R₉, -NR₈COR₉, -NR₈COOR₉, -NR₈SO₂R₉, -SO₂R₈, -SO₃R₈, -SO₂NR₈R₉ oder -N=N-R₈ bedeuten; und R₆ bis R₉ unabhängig voneinander C₁-C₄-Alkyl, Phenyl oder 4-Tolyl sind.

2. Perylenamidinimide nach Anspruch 1, worin R₁-CH(R₁₀)₂ bedeutet, und R₁₀ C₄-C₁₈-Alkyl, vorzugsweise C₆-C₁₀-Alkyl ist, oder worin R₁ einen Rest der Formel II mit R gleich tert-Butyl bedeutet.

3. Perylenamidinimide nach Anspruch 2, worin R₁ 2,5-Di-tert-butylphenyl oder -CH(R₁₀)₂ bedeutet und R₁₀ ein geradkettiger Rest, vorzugsweise n-Hexyl, n-Heptyl, n-Octyl, n-Nonyl oder n-Decyl, ist.

4. Perylenamidinimide nach einem der Ansprüche 1 bis 3, worin A 1,2-Cyclopentylen, 1,2-Cyclohexylen, 1,2-Phenylen, 2,3- oder 1,8-Naphthylen, 2,3- oder 3,4-Pyridylen, 9,10-Phenanthrylen, 1,2-Anthrachinolylen oder einen zweiwertigen Rest der Formel III, IV oder V nach Anspruch 1 bedeutet.

5. Perylenamidinimide nach einem der Ansprüche 1 bis 4, worin A 1,2-Phenylen, 2,3-oder 1,8-Naphthylen, 9,10-Phenanthrylen, 1,2-Anthrachinolylen oder ein zweiwertiger Rest der Formel III, VI oder VII ist

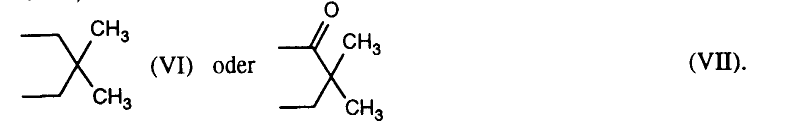

6. Perylenamidinimide nach einem der Ansprüche 1 bis 5, worin R₂ bis R₅ unabhängig voneinander H, Halogen, Nitro, -OR₈, -NR₈R₉, -NR₈COR₉, -SO₃R₈ oder -SO₂NR₈R₉ bedeuten und R₈ und R₉ unabhängig voneinander vorzugsweise Methyl, Phenyl oder 4-Tolyl sind.

7. Perylenamidinimide nach einem der Ansprüche 1 bis 6, worin R₂ bis R₅ H oder jeweils den gleichen Substituenten bedeuten.

8. Perylenamidinimide nach einem der Ansprüche 1 bis 7, worin mindestens zwei, vorzugsweise alle Reste R₂ bis R₅ H bedeuten.

9. Verfahren zur Herstellung von Perylenamidinimiden nach Anspruch 1 durch Umsetzung eines Perylen-3,4:9,10-tetracarbonsäure-monoanhydrids-monoimids der Formel VIII 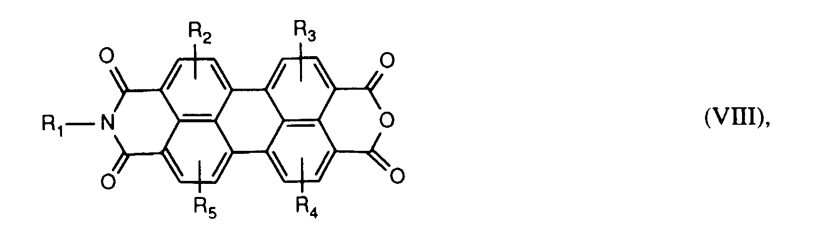 mit einem primären Diamin der Formel IX
H₂N-A-NH₂ (IX),
wobei in den Formeln VIII und IX R₁ und A die in Anspruch 1 angegebene Bedeutung haben, mit der Massgabe, dass A nicht für einen Rest der Formel III steht.

10. Verfahren zur Herstellung von Perylenamidinimiden nach Anspruch 1, worin A für einen Rest der Formel III steht, durch Umsetzung eines gegebenenfalls substituierten Imidazols mit einem Perylen-3,4:9,10-tetracarbonsäure-monoanhydrid-monoimid der Formel VIII 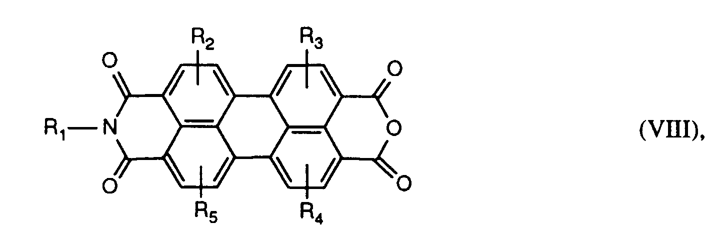 wobei R₁in Formel VIII die in Anspruch 1 angegebene Bedeutung hat.

11. In der Masse gefärbtes hochmolekulares organisches Material enthaltend als Farbmittel ein Perylenamidinimid nach Anspruch 1.

12. Verwendung der Perylenamidinimide nach Anspruch 1 als Fluoreszenzfarbstoffe, insbesondere in Chemilumineszensystemen, in optischen Lichtsammelsystemen, in Fluoreszenz-Solarkollektoren, in Fluoreszenz-aktivierten Displays oder in der "Single-Molecule Spectroscopy".

13. Verwendung der Perylenamidinimide nach Anspruch 1 als photoleitfähige Substanzen in der Elektrophotographie.

## Claims

1. A perylene amidine imide of the formula I 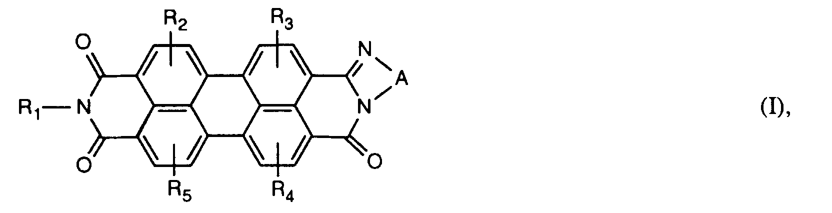 where R₁ is a secondary C₇-C₄₁alkyl radical or a radical of the formula II 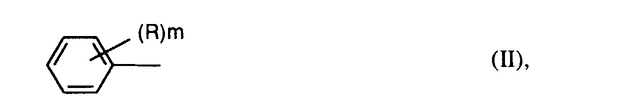 where R is a branched C₃-C₈alkyl radical and m is 1, 2 or 3;
A is C₅-C₇cycloalkylene, phenylene, naphthylene, pyridylene, a more highly fused aromatic carbocyclic or heterocyclic radical or a bivalent radical of the formula III, IV or V 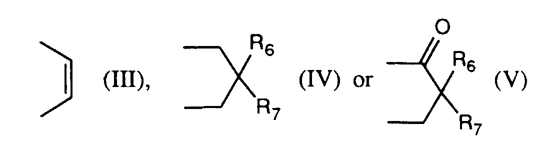 and R₁ and A may each be substituted by halogen, alkyl, cyano or nitro;
R₂ to R₅ are each independently of the others hydrogen, alkyl, aryl, hetaryl, halogen, cyano, nitro, -OR₈, -COR₈, -COOR₈, -OCOR₈, -CONR₈R₉, -OCONR₈R₉, -NR₈R₉, -NR₈COR₉, -NR₈COOR₉, -NR₈SO₂R₉, -SO₂R₈, -SO₃R₈, -SO₂NR₈R₉ or -N=N-R₈; and R₆ to R₉ are each independently of the others C₁-C₄alkyl, phenyl or 4-tolyl.

2. A perylene amidine imide according to claim 1, wherein R₁ is -CH(R₁₀)₂ and R₁₀ is C₄-C₁₈alkyl, preferably C₆-C₁₀alkyl, or wherein R₁ is a radical of the formula II where R is tert-butyl.

3. A perylene amidine imide according to claim 2, wherein R₁ is 2,5-di-tert-butylphenyl or -CH(R₁₀)₂ and R₁₀ is a straight-chain radical, preferably n-hexyl, n-heptyl, n-octyl, n-nonyl or n-decyl.

4. A perylene amidine imide according to any one of claims 1 to 3, wherein A is 1,2-cyclopentylene, 1,2-cyclohexylene, 1,2-phenylene, 2,3- or 1,8-naphthylene, 2,3- or 3,4-pyridylene, 9,10-phenanthrylene, 1,2-anthraquinolylene or a bivalent radical of the formula III, IV or V as set forth in claim 1.

5. A perylene amidine imide according to any one of claims 1 to 4, wherein A is 1,2-phenylene, 2,3- or 1,8-naphthylene, 9,10-phenanthrylene, 1,2-anthraquinolylene or a bivalent radical of the formula III, VI or VII

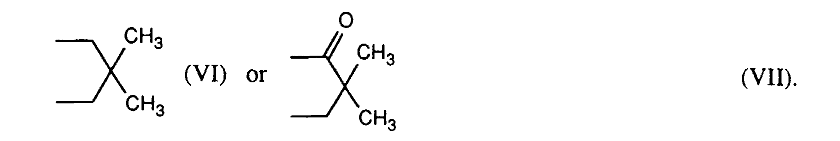

6. A perylene amidine imide according to any one of claims 1 to 5, wherein R₂ to R₅ are each independently of the others hydrogen, halogen, nitro, -OR₈, -NR₈R₉, -NR₈COR₉, -SO₃R₈ or -SO₂NR₈R₉ and R₈ and R₉ are each independently of the other preferably methyl, phenyl or 4-tolyl.

7. A perylene amidine imide according to any one of claims 1 to 6, wherein R₂ to R₅ are each hydrogen or the same substituent.

8. A perylene amidine imide according to any one of claims 1 to 7, wherein at least two, preferably all, radicals R₂ to R₅ are hydrogen.

9. A process for preparing a perylene amidine imide according to claim 1 by reacting a perylene-3,4:9,10-tetracarboxylic monoanhydride monoimide of the formula VIII 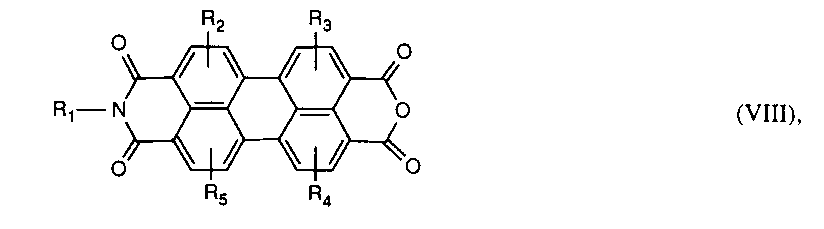 with a primary diamine of the formula IX
H₂N-A-NH₂ (IX),
where, in the formulae VIII and IX, R₁ and A are each as defined in claim 1, with the proviso that A is not a radical of the formula III.

10. A process for preparing a perylene amidine imide according to claim 1 where A is a radical of the formula III by reacting a substituted or unsubstituted imidazole with a perylene-3,4:9,10-tetracarboxylic monoanhydride monoimide of the formula VIII 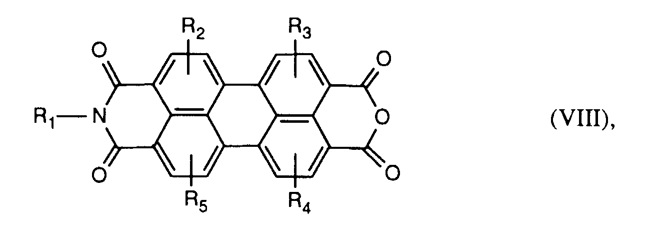 where R₁, in the formula VIII, is as defined in claim 1.

11. A mass coloured macromolecular organic material including as colorant a perylene amidine imide according to claim 1.

12. The use of the perylene amidine imide of claim 1 as a fluorescent dye, especially in chemiluminescence systems, in optical light collection systems, in fluorescence-based solar collectors, in fluorescence-activated displays or in single-molecule spectroscopy.

13. The use of the perylene amidine imide of claim 1 as a photoconductor in electrophotography.

## Revendications

1. Pérylène-amidinimides de formule I 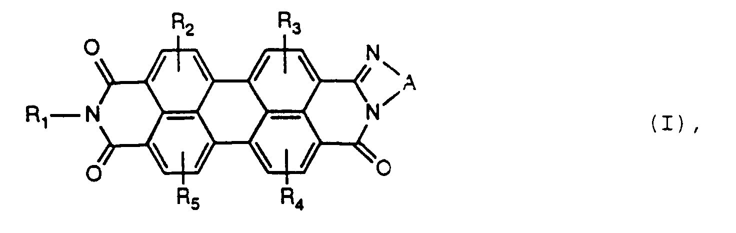 où R₁ représente un reste alkyle secondaire en C₇-C₄₁ ou un reste de formule II 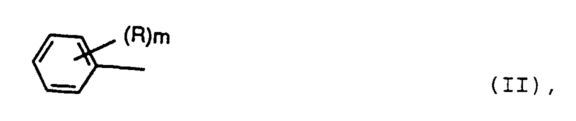 où R représente un reste alkyle en C₃-C₈ à chaîne ramifiée et m vaut 1, 2 ou 3 ;
A représente un cycloalkylène en C₅-C₇, un phénylène, un naphtylène, un pyridylène, un reste hétérocyclique ou carbocyclique aromatique à degré de condensation supérieur ou un reste bivalent de formule III, IV ou V 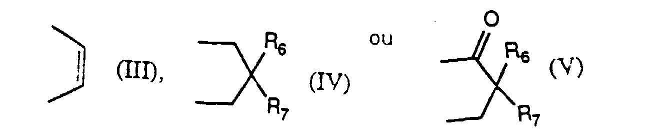 R₁, tout comme A, pouvant être substitué par un un atome d'halogène, un groupe alkyle, un groupe cyano ou un groupe nitro ;
R₂ à R₅ représentent, indépendamment l'un de l'autre, un atome d'hydrogène, un groupe alkyle, un groupe aryle, un groupe hétéroaryle, un atome d'halogène, un groupe cyano, un groupe nitro, -OR₈, -COR₈, -COOR₈, -OCOR₈, -CONR₈R₉, -OCONR₈R₉, -NR₈R₉, -NR₈COR₉, -NR₈COOR₉, -NR₈SO₂R₉, -SO₂R₉, -SO₃R₉, -SO₂NR₈R₉ ou -N=N-R₈ ; et R₆ à R₉ représentent, indépendamment l'un de l'autre, un groupe alkyle en C₁-C₄, un groupe phényle ou un groupe 4-tolyle.

2. Pérylène-amidinimides selon la revendication 1, où R₁ représente -CH(R₁₀)₂, et R₁₀ représente un groupe alkyle en C₄-C₁₈, de préférence un groupe alkyle en C₆-C₁₀, où R₁ représente un reste alkyle de formule II avec R représente un groupe tertio-butyle.

3. Pérylène-amidinimides selon la revendication 1, où R₁ représente un groupe 2,5-di-tertio-butylphényle ou -CH(R₁₀)₂ et R₁₀ représente un reste à chaîne linéaire, de préférence, les groupes n-hexyle, n-heptyle, n-octyle, n-nonyle ou n-décyle.

4. Pérylène-amidinimides selon l'une des revendications 1 à 3, où A représente les groupes 1,2-cyclopentylène, 1,2-cyclohexylène, 1,2-phénylène, 2,3-ou 1,8-naphtylène, 2,3- ou 3,4-pyridylène, 9,10-phénanthrylène, 1,2-anthraquinolylène ou un reste bivalent de formule III, IV ou V selon la revendication 1.

5. Pérylène-amidinimides selon l'une des revendications 1 à 4, où A représente les groupes 1,2-phénylène, 2,3- ou 1,8-naphtylène, 9,10- phénanthrylène, 1,2-anthraquinolylène ou un reste bivalent de formule III, VI ou VII

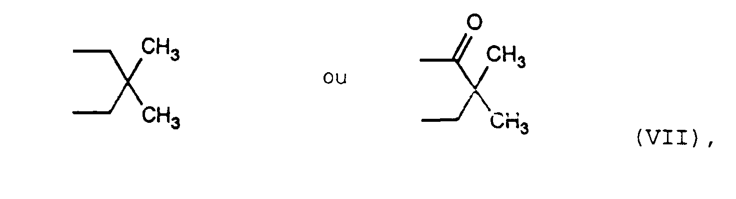

6. Pérylène-amidinimides selon l'une des revendications 1 à 5, où R₂ à R₅ représentent, indépendamment l'un de l'autre, un atome d'hydrogène, un atome d'halogène, un groupe nitro, -OR₈, -NR₈R₉, -NR₈COR₉, -SO₃R₈ ou -SO₂NR₈R₉, et R₈ et R₉ représentent, indépendamment l'un de l'autre, de préférence les groupes méthyle, phényle ou 4-tolyle.

7. Pérylène-amidinimides selon l'une des revendications 1 à 6, où R₂ à R₅ représentent un atome d'hydrogène ou chacun le même substituant.

8. à chaque fois comme substituant selon l'une des revendications 1 à 7, où au moins deux, de préférence tous les restes R₂ à R₅ représentent un atome d'hydrogène.

9. Procédé de préparation de pérylène-amidinimides selon la revendication 1, par réaction d'un monoanhydride-monoimide d'acide pérylène-3,4:9, 10-tétra-carboxylique de formule VIII 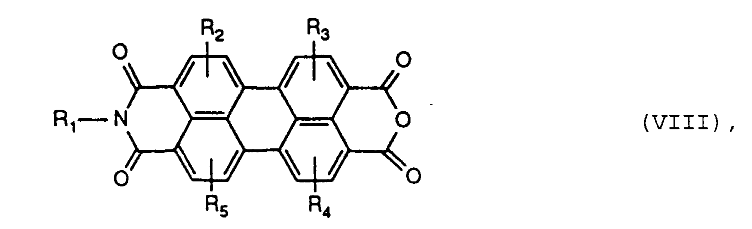 avec une diamine primaire de formule IX
H₂N-A-NH₂ (IX),
où, dans les formules VIII et IX, R₁ et A possèdent les significations données à la revendication 1, à la condition que A ne représente pas un reste de formule III.

10. Procédé de préparation de pérylène-amidinimides selon la revendication 1, où A représente un reste de formule III, par réaction d'un imidazole éventuellement substitué avec un monoanhydride-monoimide d'acide pérylène-3,4:9,10-tétracarboxylique de formule VIII 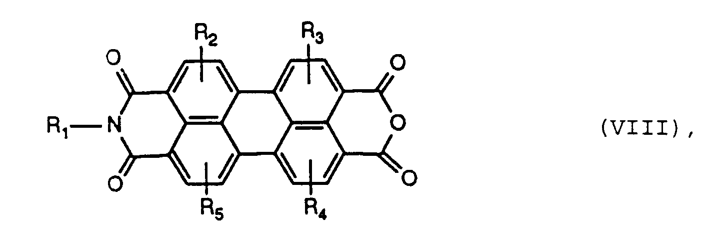 où R₁ dans la formule VIII possède la signification donnée de la revendication 1.

11. Matière organique de haut poids moléculaire pigmentée en masse, contenant comme colorant un pérylène-amidinimide de selon la revendication 1.

12. Utilisation de pérylène-amidinimides selon la revendication 1 comme colorants fluorescents, en particulier dans des systèmes de chimioluminescence, dans des systèmes optiques d'accumulation de lumière, dans des collecteurs solaires à fluorescence, dans des afficheurs fluorescents activés ou dans la "Single-Molecule Spectroscopy".

13. Utilisation de pérylène-amidinimides selon la revendication 1, comme substances photoconductrices dans l'électrophotographie.
